# EUROPÄISCHE PATENTSCHRIFT

(11) **EP 3 703 795 B1**
(45) Veröffentlichungstag und Bekanntmachung des Hinweises auf die Patenterteilung: **20.05.2026**
(21) Anmeldenummer: 18793020.1
(22) Anmeldetag: 18.10.2018
(51) Int. Cl.: A61M 5/315

(54) **STROMVERSORGUNG ZUSATZMODUL**
POWER SUPPLY ADD-ON MODULE
MODULE COMPLÉMENTAIRE D'ALIMENTATION ÉLECTRIQUE

(30) Priorität: 31.10.2017 CH 13102017
(43) Veröffentlichungstag der Anmeldung: 09.09.2020
(73) Patentinhaber: Ypsomed AG, 3401 Burgdorf (CH)
(72) Erfinder: GENTZ, Michael, 3400 Burgdorf (CH)
(74) Vertreter: Meier Obertüfer, Jürg
(86) Internationale Anmeldenummer: PCT/IB2018/058070
(87) Internationale Veröffentlichungsnummer: WO 2019/086992

(56) Entgegenhaltungen:
- WO-A1-2017/129323
- US-A1- 2004 204 673
- US-A1- 2009 069 749
- US-A1- 2012 184 907
- US-A1- 2015 202 375
- US-A1- 2015 273 163
- US-A1- 2016 022 539

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf die Stromversorgung eines mit einem Einweginjektor lösbar verbindbaren Mehrwegzusatzmoduls zum Erkennen von Injektionsereignissen im Einweginjektor.

### HINTERGRUND DER ERFINDUNG

Aus dem Stand der Technik sind Injektionssysteme bekannt, bei denen an einen Einweginjektor wie beispielsweise einen Autoinjektor zur Abgabe einer medizinischen Substanz ein wiederverwendbares elektronisches Zusatzmodul angebracht werden kann. Ein solches Zusatzmodul wird auch als Elektronikmodul, "Smart device", "Smart module" oder "Add-on" bezeichnet. Das Zusatzmodul wird vor dem Injektionsbeginn mit einem Einweginjektor verbunden. Hierzu ist das Zusatzmodul lösbar auf den Einweginjektor aufsetzbar oder aufsteckbar. Das Zusatzmodul dient dazu Vorgänge, Ereignisse und Funktionen des Einweginjektors zu erkennen, zu überwachen und erfasste Ereignisse dem Anwender zu melden. So kann beispielsweise der Patient in Echtzeit beim Injektionsvorgang durch das Zusatzmodul unterstützt werden, indem auf einer Applikation auf einem Smartphone Instruktionen für die Injektion, Vorschläge für die Injektionszeit und Handhabungsfehler mitgeteilt werden. Ferner können mittels des Zusatzmoduls visuelle und akustische Rückmeldung für spezielle Ereignisse wie das erfolgreiche Abschliessen einer Injektion ausgegeben werden. Das Zusatzmodul kann zudem Informationen über getätigte Injektionen aufzeichnen und Auswertungen vornehmen.

Die Patentanmeldung US 2016/243318 A offenbart ein Zusatzmodul für einen Autoinjektor. Das Zusatzmodul kann in einen bestehenden Autoinjektor eingesetzt oder aussen an diesem befestigt werden. Es umfasst einen Sensor zum Erfassen von Umgebungszuständen wie beispielsweise Temperatur, Druck, Bewegung, Orientierung oder elektromagnetische Strahlung. Weiter kann das Zusatzmodul den Funktionszustand des Autoinjektors erkennen. Die Sensoren können die Umgebungszustände je nach verfügbarer elektrischer Energie stündlich, täglich oder wöchentlich aufzeichnen. Das Zusatzmodul umfasst eine Batterie, um die Elektronik mit Energie zu versorgen.

Die US 2012/0184907 offenbart eine Infusionspumpe, welche eine Pumpeneinheit und eine Basiseinheit umfasst, die lösbar miteinander verbindbar sind. Die Pumpeneinheit, welche wiederverwendbar ist, umfasst eine aufladbare Batterie. Die Pumpeneinheit ist auf ein Batterieladegerät aufsetzbar, um die aufladbare Batterie zu laden. Dabei kontaktieren elektrische Kontakte der Pumpeneinheit elektrische Kontakte des Batterieladegeräts. In einer alternativen Ausführung oder zusätzlich kann die wiederaufladbare Batterie in der Pumpeneinheit auch durch einen Energiespeicher in der Basiseinheit aufgeladen werden, wenn die Pumpeneinheit mit der Basiseinheit verbunden ist.

US 2004/204673 offenbart eine Infusionspumpe mit einer wiederverwendbaren Einheit und einer Einwegeinheit. Die wiederverwendbare Einheit umfasst einen Kondensator als Energiespeicher, welcher durch eine Batterie der Einwegeinheit aufgeladen werden kann, wenn die beiden Einheiten miteinander verbunden sind. Die Einwegeinheit umfasst Sensoren, um den Fluidfluss oder um den Betrieb der wiederverwendbaren Einheit zu überwachen. Auch die wiederverwendbare Einheit umfasst eine Sensoreinheit, um den Fluidfluss in der Pumpe zu messen oder um Okklusion zu erkennen.

US 2009/0069749 beschreibt eine Infusionspumpe, welche eine Einweg-Pumpeneinheit und eine mehrfach verwendbare Steuerungseinheit umfasst. Die Steuerungseinheit umfasst eine Steuerung mit einem Okklusionssensor. Weiter umfasst die Steuerungseinheit eine wiederaufladbare Batterie, die durch eine Batterie in der Pumpeneinheit aufladbar ist, wenn die Steuerungseinheit mit der Pumpeneinheit verbunden ist.

WO 2017/129323 offenbart eine gedruckte Batterie für eine gekrümmte Oberfläche wie beispielweise eine Oberfläche eines Autoinjektors oder einer Flasche. Die Batterie umfasst eine Trägerfolie, eine erste Elektrodenschicht zur Ausbildung einer Kathode und eine zweite Elektrodenschicht zur Ausbildung einer Anode. Zum Abgreifen einer Spannung weist die Batterie zwei Anschlussflächen auf.

US 2015/0202375 beschreibt einen Einweg-Injektionspen mit einem aufsteckbaren Zusatzmodul. Das Zusatzmodul umfasst eine Sensoreinheit, welche eine Bewegung der Antriebseinrichtung überwachen kann zum Erkennen einer mit dem Injektionspen verabreichten Dosis. Weiter umfasst das Zusatzmodul eine Batterie, um die Sensoreinheit im Zusatzmodul mit elektrischer Energie zu versorgen.

US 2015/0273163 offenbart einen Einweg-Injektionspen, welcher eine elektrische Schaltung und eine Sensoreinheit umfasst, mit welcher Injektionsereignisse aufgezeichnet werden können. Die im Injektionspen generierten Daten werden an einen externen Empfänger übermittelt. Eine Kappe, welche eine Batterie umfasst, kann auf den einstechseitigen Bereich des Injektionspen aufgesetzt werden. Die Batterie in der Kappe lädt eine wiederaufladbare Batterie im Injektionspen, wenn die Kappe aufgesetzt ist.

Eine weitere Ausführung eines Zusatzmoduls beschreibt die Patentanmeldung US 2016/213853 A. Diese offenbart ein Detektionsmodul für einen Injektor, welches einen Bewegungssensor und einen Vibrationssensor beinhaltet zum Erfassen einer Bewegung oder einer Vibration des Injektors. Mit diesen Sensoren kann erfasst werden, welche Dosis der Benutzer für die Injektion gewählt hat. Das Detektionsmodul kann an der Aussenseite eines bestehenden Injektors angeklemmt werden. Eine Batterie im Detektionsmodul versorgt die Sensoren mit elektrischer Energie.

Bei solchen Systemen werden üblicherweise Knopfbatterien oder herkömmliche zylinderförmige Batterien eingesetzt. Für Spezialanwendungen sind jedoch auch sogenannte gedruckte Batterien bekannt. Diese können in mehreren Drucklagen hergestellt werden und zeichnen sich durch ihre sehr dünne Bauweise aus. Die Patentanmeldung WO 17129323 A1 offenbart eine solche gedruckte Batterie für eine gekrümmte Oberfläche wie beispielweise eine Oberfläche eines Autoinjektors oder einer Flasche. Die Batterie umfasst eine Trägerfolie, eine erste Elektrodenschicht zur Ausbildung einer Kathode und eine zweite Elektrodenschicht zur Ausbildung einer Anode. Zum Abgreifen einer Spannung weist die Batterie zwei Anschlussflächen auf.

Im technischen Gebiet der Infusionssysteme sind Vorrichtungen bekannt, die eine Einwegkomponente und eine wiederverwendbare Komponente umfassen. Die Einwegkomponente beinhaltet dabei Verbrauchsartikel wie ein vorgefülltes Reservoir für die abzugebende Substanz, eine Kanüle und Klebepflaster, um die Einwegkomponente auf der Haut eines Patienten ankleben zu können. Die Mehrwegkomponente umfasst vorzugsweise Komponenten, welche für mehrere Abgabevorgänge verwendet werden können wie beispielsweise eine Pumpe, eine Energieversorgung und eine Steuereinheit. Ein solches Infusionssystem beschreibt beispielsweise die Patentanmeldung WO 2011/015659 A1. Dieses System umfasst eine auf der Haut des Patienten anbringbare Einwegkomponente, welche eine Basisplatte, eine Kartusche, eine Einwegbatterie und eine Kanüle beinhaltet. Eine Mehrwegkomponente, welche lösbar mit der Einwegkomponente verbindbar ist, umfasst einen Motor, eine Pumpe mit Steuerung und eine wiederaufladbare Batterie. Wenn die Mehrwegkomponente mit der Basisplatte verbunden ist, lädt die Einwegbatterie der Einwegkomponente die wiederaufladbare Batterie in der Mehrwegkomponente auf.

Im Unterschied zu den oben erwähnten Infusionssystemen ist der zur Verfügung stehende Platz für die Energieversorgung bei Zusatzmodulen für Injektoren stark eingeschränkt. Bei den aus dem Stand der Technik bekannten Zusatzmodulen mit einer auswechselbaren nicht aufladbaren Knopfbatterie ist die Energiespeicherkapazität sehr beschränkt. Das bedingt eine aufwändige Stromspartechnologie im Zusatzmodul, um die zur Verfügung stehende Energie so gut wie möglich nutzen zu können. Eine auswechselbare Batterie hingegen bedingt, dass der Benutzer den Ladezustand der Batterie überwacht und die Batterie rechtzeitig auswechselt. Zudem stellt eine auswechselbare Batterie erhöhte Anforderungen an die Gehäusemechanik, damit die Batterie einfach entnommen und wieder eingesetzt werden kann.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Unter den Begriffen "Injektionssystem" oder "Injektor" wird in der vorliegenden Beschreibung eine Vorrichtung verstanden, bei der die Injektionsnadel nach Abgabe der medizinischen Substanz aus dem Gewebe des Patienten entfernt wird. Somit verbleibt bei einem Injektionssystem oder bei einem Injektor im Unterschied zu einem Infusionssystem die Injektionsnadel nicht permanent bzw. über einen längeren Zeitraum von mehreren Stunden im Patienten.

Ein Einweginjektor ist ein Injektor, welcher dazu verwendet wird, die in einer nicht nachfüllbaren und nicht austauschbaren Karpule befindliche medizinische Substanz subkutan zu injizieren. Ist die zur Injektion vorgesehene Menge der medizinischen Substanz in einem oder in mehreren Injektionsvorgängen injiziert worden, wird der Einweginjektor ausgewechselt. Die Karpule im Einweginjektor kann nicht ausgetauscht werden. Demgegenüber ist bei einem Mehrweginjektor die Karpule mit der medizinischen Substanz austauschbar. Die Zusatzmodule stellen eine Mehrwegkomponente dar und werden üblicherweise von einem Einweginjektor zum nächsten übertragen.

### DARSTELLUNG DER ERFINDUNG

Es ist eine Aufgabe der Erfindung, eine zuverlässige Stromversorgung für ein Zusatzmodul für einen Einweginjektor zu schaffen, wobei das Zusatzmodul einfach und kompakt konstruierbar sein soll. Die Erfindung ist ausschließlich durch die unabhängigen Ansprüche 1 und 8 definiert. Bevorzugte Ausführungsformen sind in den abhängigen Ansprüchen definiert.

Die Aufgabe wird durch ein Injektionssystem gelöst, welches einen Einweginjektor und ein lösbar mit dem Einweginjektor verbindbares Mehrwegzusatzmodul, welches in einem verbundenen Zustand Injektionsereignisse des Einweginjektors erkennen kann, umfasst. Dabei umfasst der Einweginjektor einen Energieträger und eine Übertragungseinheit zum Übertragen von elektrischer Energie vom Energieträger. Das Mehrwegzusatzmodul umfasst einen aufladbaren Energiespeicher und eine Ladeeinheit zum Aufnehmen von elektrischer Energie für den Energiespeicher. Die Übertragungseinheit und die Ladeeinheit sind so ausgebildet, dass im verbundenen Zustand der Energiespeicher mit elektrischen Energie vom Energieträger aufladbar ist.

Vor dem Injektionsvorgang wird der Einweginjektor mit dem Mehrwegzusatzmodul lösbar verbunden. Hierzu wird das Mehrwegzusatzmodul vorzugsweise auf den Einweginjektor aufgeschoben oder aufgesteckt. Dabei werden die Übertragungseinheit und die Ladeeinheit zueinander hin bewegt, so dass über die Übertragungseinheit und die Ladeeinheit elektrische Energie zwischen dem Energieträger des Einweginjektors und dem Energiespeicher des Mehrwegzusatzmoduls übertragbar ist. Die elektrische Energie kann zwischen Übertragungseinheit und Ladeeinheit über einen physischen Kontakt oder kontaktlos übertragen werden.

Im verbundenen Zustand kann das Mehrwegzusatzmodul die Injektionsereignisse, Vorgänge und Funktionen im Einweginjektor erkennen, laufend überwachen und dem Anwender visuelle und akustische Meldungen ausgeben. Bevorzugt werden beim Verbinden des Mehrwegzusatzmoduls mit dem Einweginjektor die elektrischen Verbraucher des Mehrwegzusatzmoduls aktiviert. Alternativ dazu besteht auch die Möglichkeit, dass manuell durch den Benutzer ein Schalter betätigt werden muss zum Aktivieren der Verbraucher im Mehrwegzusatzmodul.

Durch die Möglichkeit elektrische Energie zwischen Übertragungseinheit und Ladeeinheit zu übertragen, kann der Energiespeicher im Mehrwegzusatzmodul durch den Energieträger im Einweginjektor aufgeladen werden. Das bedeutet, dass keine weitere Stromversorgungseinrichtung für die elektrischen Verbraucher im Mehrwegzusatzmodul vonnöten ist. Insbesondere muss das Mehrwegzusatzmodul nicht an eine externe Stromquelle angeschlossen werden, um die elektrischen Verbraucher im Mehrwegzusatzmodul mit elektrischer Energie zu speisen oder um den Energiespeicher aufzuladen. In einer bevorzugten Ausführung ist der Energiespeicher exklusiv durch den Energieträger des Einweginjektors aufladbar. Das heisst, das Mehrwegzusatzmodul weist keinen anderen Anschluss für eine Stromversorgung auf. Das vereinfacht die Konstruktion und die Bedienung des Mehrwegzusatzmoduls.

Vorzugsweise erfolgt der Ladevorgang des Energiespeichers während des Betriebs des Mehrwegzusatzmoduls, das heisst, während dem das Mehrwegzusatzmodul die Injektionsereignisse und Vorgänge im Einweginjektor detektiert und überwacht. In einer alternativen Ausführung besteht auch die Möglichkeit, dass das Mehrwegzusatzmodul in einem ersten Schritt seinen Energiespeicher durch den Energieträger auflädt und anschliessend in einem zweiten Schritt seinen Betrieb aufnimmt. Ferner besteht die Möglichkeit, dass das Mehrwegzusatzmodul auch nach dem Injektionsvorgang auf dem Einweginjektor verbleibt, um den Energiespeicher weiter aufzuladen und eine möglichst vollständige Entleerung des Energieträgers zu ermöglichen.

Üblicherweise ist das Mehrwegzusatzmodul nur kurze Zeit nicht mit einem Einweginjektor verbunden, da in der Regel nach dem Entfernen eines zu ersetzenden Einweginjektors im Anschluss das Mehrwegzusatzmodul an einen neuen Einweginjektor angeschlossen wird. Dadurch wird keine grosse Kapazität des Energiespeichers im Mehrwegzusatzmodul benötigt. Das ermöglicht den Einsatz eines Energiespeichers von kompakter Baugrösse, so dass das Mehrwegzusatzmodul kompakt konstruiert werden kann.

In einer alternativen Ausführung kann das Mehrwegzusatzmodul über einen längeren Zeitraum, beispielsweise mehrere Tage, mit dem Einweginjektor verbunden sein, damit während dieser Zeit der Energieträger den Energiespeicher laden und/oder die elektrischen Verbraucher im Mehrwegzusatzmodul mit elektrischer Energie versorgen kann.

Ferner kann mit dem erfindungsgemässen Injektionssystem der Energiespeicher im Mehrwegzusatzmodul automatisch nachgeladen werden ohne dass der Anwender den Ladungszustand des Energiespeichers überwachen muss oder manuell eine Nachladung vornehmen muss. Das erhöht die Benutzerfreundlichkeit des Injektionssystems.

Der Energiespeicher im Mehrwegzusatzmodul ist vorzugsweise als Akkumulator ausgebildet. Aus dem Stand der Technik sind unterschiedliche Akkumulatortypen bekannt wie beispielsweise Lithium-Ionen-Akkumulatoren, Lithium-Polymer-Akkumulator, Nickel-Metallhydrid-Akkumulatoren oder Nickel-Cadmium-Akkumulatoren. In einer alternativen Ausführung kann der Energiespeicher als Kondensator, beispielsweise als Keramikkondensator, Elektrolytkondensator oder als Superkondensator, ausgebildet sein. Zudem kann der Energiespeicher auch aus einer Kombination von unterschiedlichen Akkumulatortypen bestehen, zum Beispiel aus einer Kombination eines Lithium-Polymer-Akkumulators und eines Superkondensators.

Bevorzugt ist der Energieträger im Einweginjektor als Einwegbatterie ausgebildet, welche in einem einmaligen Vorgang entladbar ist. Vorzugsweise ist der Energieträger auf einer Aussenseite des Einweginjektors angeordnet. Der Energieträger kann jedoch auch innerhalb eines Gehäuses des Einweginjektors angeordnet sein.

In einer Ausführung der Erfindung kann das Injektionssystem dazu verwendet werden, neben der Übertragung von elektrischer Energie zwischen dem Mehrwegzusatzmodul und dem Einweginjektor zusätzlich Signale zwischen dem Mehrwegzusatzmodul und dem Einweginjektor zu übermitteln, um eine Datenübertragung zu ermöglichen. Hierzu umfassen das Mehrwegzusatzmodul und der Einweginjektor je eine Kommunikationsschnittstelle. So können mittels der Signale zum Beispiel Informationen zum Typ des Einweginjektors, zur verwendeten medizinischen Substanz oder zum Injektionsstart und -ende über die Kommunikationsschnittstelle vom Einweginjektor zum Mehrwegzusatzmodul übermittelt werden. In diesem Fall muss der Einweginjektor einen Sensor zum Erfassen der zu übertragenden Signalen beinhalten. Alternativ oder zusätzlich können auch Signale vom Mehrwegzusatzmodul zum Einweginjektor übertragen werden. Das Übertragen der Signale kann beispielsweise mittels "radio-frequency identification" (RFID) erfolgen. In diesem Fall umfasst die eine Kommunikationsschnittstelle einen RFID-Transponder, der einen Code enthält und die andere Kommunikationsschnittstelle umfasst ein Lesegerät zum Auslesen des Codes. Alternativ dazu, ist eine Übertragung mittels Bluetooth möglich.

In einer bevorzugten Ausführungsform der Erfindung umfasst das Mehrwegzusatzmodul ferner eine Kommunikationseinheit zur drahtlosen Kommunikation mit einem mobilen Gerät, beispielsweise einem Mobiltelefon oder Smartphone, und/oder einem optischen, akustischen, oder taktilen Zustandsanzeiger. Die erfassten Injektionsereignisse können dadurch an einem Anzeigegerät dem Benutzer ersichtlich gemacht werden.

In einer bevorzugten Ausführung des erfindungsgemässen Injektionssystems umfasst die Übertragungseinheit des Einweginjektors eine erste Kontaktplatte und die Ladeeinheit des Mehrwegzusatzmoduls eine zweite Kontaktplatte zum elektrischen Verbinden mit der ersten Kontaktplatte. Dabei ist die erste Kontaktplatte elektrisch mit dem Energieträger und die zweite Kontaktplatte elektrisch mit dem Energiespeicher verbunden, so dass elektrische Energie vom Energieträger über die erste und die zweite Kontaktplatte zum aufladbaren Energiespeicher im Mehrwegzusatzmodul übertragbar ist, wenn der Einweginjektor mit dem Mehrwegzusatzmodul verbunden ist. Das bedeutet, in diesem verbundenen Zustand berührt die erste Kontaktplatte die zweite Kontaktplatte. Eine solche elektrische Verbindung zwischen Einweginjektor und Übertragungseinheit ist konstruktiv einfach und kostengünstig realisierbar.

In einer bevorzugten Ausführung der Erfindung ist die erste Kontaktplatte auf einer Aussenfläche des Einweginjektors und die zweite Kontaktplatte auf einer Innenfläche des Mehrwegzusatzmoduls angeordnet, wobei die Innenfläche der Aussenfläche gegenüberliegt, wenn der Einweginjektor mit dem Mehrwegzusatzmodul verbunden ist.

In einer alternativen Ausführung der Erfindung umfasst die Übertragungseinheit einen Oszillator und eine erste Spule zum Erzeugen eines magnetischen Wechselfeldes und die Ladeeinheit umfasst eine zweite Spule zum Empfangen einer Wechselspannung. Dadurch kann die elektrische Energie kontaktlos und induktiv vom Energieträger zum aufladbaren Energiespeicher übertragen werden. Die Ladeeinheit umfasst vorzugsweise zudem einen Gleichrichter, um die in der zweiten Spule induzierte Wechselspannung gleichzurichten, so dass der Energiespeicher mit Gleichspannung geladen werden kann.

Bevorzugt werden bei der Übertragungseinheit und bei der Ladeeinheit je ein vorgefertigter, aus dem Stand der Technik bekannter und frei erhältlicher Schaltkreis eingesetzt, welcher das kontaktlose Übertragen von elektrischer Energie ermöglicht.

Durch die kontaktlose Energieübertragung kann der mechanische Verschleiss an Kontaktstellen vermieden werden. In einer bevorzugten Ausführung ist die erste Spule in einem hinteren, dem einstechseitigen Ende des Einweginjektors abgewandten Bereich in einem Gehäuse des Einweginjektors integriert. Entsprechend ist vorzugsweise die zweite Spule so im Mehrwegzusatzmodul integriert, dass sich im verbundenen Zustand die erste Spule und die zweite Spule gegenüberliegen, damit die elektrische Energie möglichst effizient übertragbar ist.

In einer weiteren Ausführung der Erfindung umfasst die Übertragungseinheit einen Oszillator und ein erstes Element und die Ladeeinheit umfasst ein zweites Element, wobei das erste und das zweite Element einen Kondensator bilden. Dabei wird der das erste Element mit Wechselspannung gespeist, welche durch den Oszillator erzeugt wird, so dass eine kapazitive Kopplung zwischen dem ersten und dem zweiten Element erfolgt. Dadurch kann eine kontaktlose und kapazitive Übertragung der elektrischen Energie vom Energieträger zum aufladbaren Energiespeicher ermöglicht werden. Im Anschluss an das zweite Element erfolgt vorzugsweise eine Gleichrichtung der Wechselspannung, so dass der Energiespeicher mit Gleichspannung geladen werden kann. Das erste Element ist mit Vorteil in einem hinteren, dem einstechseitigen Ende des Einweginjektors abgewandten Bereich im Gehäuse des Einweginjektors integriert. Entsprechend ist das zweite Element im Mehrwegzusatzmodul derart angeordnet, dass es im verbundenen Zustand nahe am ersten Element ist, so dass die elektrische Energie möglichst effizient und mit wenig Streuverlusten übertragbar ist.

In einer anderen Ausführung umfasst die Übertragungseinheit eine Lichtquelle zum Erzeugen eines Lichtstrahls mit optischer Leistung und die Ladeeinheit umfasst einen Photodetektor zur Umwandlung der optisch übertragenen Leistung in elektrische Leistung. Dabei kann die Lichtquelle beispielsweise durch eine Leuchtdiode (LED) gebildet werden, welche den Lichtstrahl erzeugt. Als Photodetektoren können zum Beispiel photovoltaische Zellen oder eine Photodiode eingesetzt werden. Auch bei dieser Ausführung erfolgt die Übertragung der Leistung vom Energieträger zum Energiespeicher kontaktlos.

Erfindungsgemäß weist der Einweginjektor entlang einer Längsachse eine längliche Form auf und das Mehrwegzusatzmodul weist eine Öffnung auf, in die mindestens ein Bereich des Einweginjektors in Richtung der Längsachse eingeschoben werden kann. Dabei wird vorzugsweise das Mehrwegzusatzmodul auf ein erstes Ende des Einweginjektors aufgeschoben, welches einem einstechseitigen Ende des Einweginjektors gegenüber liegt. Anschliessend wir das Mehrwegzusatzmodul mit einer Haltevorrichtung, wie beispielsweise einem Schnappverschluss in Längsrichtung am Einweginjektor fixiert. Das ermöglicht ein einfaches und rasches Verbinden von Einweginjektor mit dem Mehrwegzusatzmodul. In einer vorteilhaften Ausführung ist das Mehrwegzusatzmodul hülsenförmig ausgebildet, so dass das Mehrwegzusatzmodul im verbundenen Zustand den Einweginjektor teilweise umgibt.

In einer bevorzugten Ausführung weist zudem der Einweginjektor im Querschnitt eine im Wesentlichen viereckige Form auf und der Querschnitt der Öffnung weist entsprechend ebenfalls eine viereckige Form auf, so dass der Einweginjektor in die Öffnung einführbar ist, jedoch nicht um seine Längsachse drehbar ist. In einer alternativen Ausführung kann der Einweginjektor jedoch auch einen kreisrunden, dreieckigen oder polygonförmigen Querschnitt und die Öffnung ebenfalls einen entsprechend geformten Querschnitt aufweisen.

In einer bevorzugten Ausführungsform der Erfindung ist die Ladeeinheit entlang einer Innenfläche des Mehrwegzusatzmoduls angeordnet. Falls das Mehrwegzusatzmodul eine Öffnung umfasst, ist die Ladeeinheit mit Vorteil an einer Innenwandung der Öffnung in Umfangrichtung der Öffnung angeordnet. In diesem Fall muss der Einweginjektor, wenn er in die Öffnung eingeführt wird, nicht in eine bestimmte Position relativ zum Mehrwegzusatzmodul um seine Längsachse ausgerichtet sein, damit die Übertragungseinheit mit der Ladeeinheit zusammenwirken kann. Das vereinfacht das Verbinden des Einweginjektors mit dem Mehrwegzusatzmodul für den Benutzer. In einer alternativen Ausführung kann die Ladeeinheit auch in Längsrichtung entlang der Innenfläche der Öffnung des Mehrwegzusatzmoduls angeordnet sein. Dadurch muss der Einweginjektor beim Verbinden in Längsrichtung relativ zum Mehrwegzusatzmodul nicht exakt positioniert werden, da die Übertragungseinheit in Längsrichtung innerhalb eines Bereichs positioniert werden kann. Die Länge dieses Bereichs ist abhängig von der Länge der Ladeeinheit.

In einer vorteilhaften Ausführungsform der Erfindung ist die Übertragungseinheit entlang eines Umfangs des Einweginjektors angeordnet. Unabhängig davon, wie das Mehrwegzusatzmodul ausgebildet ist und wo die Ladeeinheit des Mehrwegzusatzmoduls angeordnet ist, muss beim Verbinden des Einweginjektors mit dem Mehrwegzusatzmodul, der Einweginjektor in Umfangrichtung nicht in einer bestimmten Position relativ zum Mehrwegzusatzmodul ausgerichtet sein, damit die Übertragungseinheit mit der Ladeeinheit zusammenwirken kann. Das erleichtert wiederum das Verbinden von Einweginjektor und Mehrwegzusatzmodul.

Bevorzugt ist der Energieträger auf einer Aussenfläche des Einweginjektors angeordnet. Das ermöglicht eine einfache Herstellung und eine einfache Kontaktierung. So können beispielsweise der Energieträger und die Übertragungseinheit nachträglich auf einen bestehenden Einweginjektor aufgebracht werden, um einen bestehenden Einweginjektor nachträglich für den Gebrauch mit einem Mehrwegzusatzmodul nachzurüsten. Bevorzugt ist der Energieträger lösbar, beispielsweise durch Aufkleben, auf einer Aussenfläche angebracht des Einweginjektors angebracht. Dadurch kann, wenn der Einweginjektor nach Gebrauch entsorgt wird, die Batterie getrennt vom Rest des Einweginjektors entsorgt und rezykliert werden.

Vorzugsweise ist der Energieträger des Einweginjektors als gedruckte Batterie ausgebildet. Gedruckte Batterien werden auch als "Printed battery", Filmbatterie oder als Etikettenbatterie bezeichnet. Eine gedruckte Batterie zeichnet sich durch ihre sehr dünne Bauweise von im Bereich von ca. 0.2 mm bis 2 mm aus. Damit ist die gedruckte Batterie wesentlich dünner als eine herkömmliche Knopfzelle. Die gedruckte Batterie umfasst bevorzugt eine Anode aus Zink und eine Kathode aus Mangandioxid. Dabei liefert die gedruckte Batterie eine Spannung zwischen 1 - 6 V und eine Nennkapazität von ca. 10 mAh bis 100 mAh. Vorzugsweise weist die gedruckte Batterie eine rechteckförmige Gestalt auf, wobei die Seitenlängen zwischen 30 und 90 mm liegen. In einer alternativen Ausführung kann die gedruckte Batterie aber auch eine polygonförmige oder kreisrunde Form aufweisen. Mit der gedruckten Batterie kann der Energieträger auf dem Einweginjektor besonders platzsparend und optisch unauffällig realisiert werden. Mit Vorteil ist die gedruckte Batterie eine Einwegbatterie, die einmalig entladen werden kann und gleichzeitig mit, aber möglicherweise getrennt von, dem Einweginjektor entsorgt werden kann.

In einer bevorzugten Ausführung ist die Batterie aus Materialien gefertigt, welche nach üblichen gesetzlichen Richtlinien keine spezielle Entsorgung benötigen. Das bedeutet, die Menge der verwendeten Metalle und Chemikalien der Batterie sind so gering, dass keine spezielle Entsorgung vonnöten ist. Die gedruckte Batterie ist somit vorzugsweise zusammen mit dem normalen Hausmüll entsorgbar. Solche Batterien werden auch als "grüne Batterien" bezeichnet. Bevorzugt weist die grüne, gedruckte Batterie nur eine Grösse zwischen 120 mm² und 200 mm² auf. Diese Grösse reicht aus, um genügend elektrische Energie bereit zu stellen, um das Mehrwegzusatzmodul betreiben zu können. Durch die kompakte Dimension kann die gedruckte Batterie einfach und optisch unauffällig auf dem Einweginjektor platziert werden.

In einer bevorzugten Ausführung des erfindungsgemässen Einweginjektors weist die gedruckte Batterie eine gekrümmte Form auf. Falls der Einweginjektor eine gekrümmte Aussenfläche aufweist und die Krümmung der Aussenfläche derjenigen der Batterie entspricht, kann die gedruckte Batterie in besonders platzsparender Weise auf die Aussenfläche des Einweginjektors angeordnet werden. Durch die dünne Bauweise der gedruckten Batterie wird das Verbinden des Einweginjektors mit dem Mehrwegzusatzmodul durch die Batterie kaum beeinträchtigt. So kann beispielsweise die gekrümmte, gedruckte Batterie in einem Bereich auf der Aussenfläche angeordnet sein, welcher sich im verbundenen Zustand in einer allenfalls vorhandenen Öffnung des Mehrwegzusatzmoduls befindet. Das bedeutet, falls das Mehrwegzusatzmodul eine Öffnung aufweist, muss diese wegen der Batterie nicht speziell gross ausgestaltet sein. Dadurch ist eine kompakte Bauweise des Mehrwegzusatzmoduls möglich. In einer anderen Ausführung weist der Einweginjektor auf einer Aussenseite eine Aussparung auf, in welche die gedruckte Batterie eingelassen ist, so dass die gedruckte Batterie die Aussenkontur des Einweginjektors nicht beeinträchtigt. In einer bevorzugten Ausführung wird die Batterie mit einer Krümmung hergestellt und anschliessend auf die gekrümmte Aussenfläche aufgebracht. Es besteht jedoch auch die Möglichkeit, dass die Batterie bei der Montage gekrümmt wird.

Erfindungsgemäß ist der Einweginjektor des erfindungsgemässen Injektionssystems ein Autoinjektor. Unter dem Begriff "Autoinjektor" wird eine Injektionsvorrichtung zum automatischen Ausschütten einer medizinischen Substanz mittels einer Antriebsvorrichtung verstanden. Vorzugsweise umfasst der Autoinjektor eine nicht elektrische Antriebsvorrichtung, welche beispielsweise mittels einer vorgespannten Feder oder mittels Gasdruck funktioniert.

Vorzugsweise umfasst das erfindungsgemässe Mehrwegzusatzmodul einen Sensor zum Erfassen von Ereignissen während einem Injektionsvorgang mit dem Einweginjektor, wobei der Sensor durch den Energiespeicher mit elektrischer Energie versorgt wird. Der Sensor kann beispielsweise als Induktiv-, Kapazitiv-, Temperatur-, Druck- oder Orientierungssensor wie auch als magnetoelastischer Sensor ausgebildet sein. Unabhängig davon, welcher Sensortyp verwendet wird, dient der Sensor dazu, Messdaten zu generieren. Anhand dieser Messdaten können in einer Auswertelektronik im Mehrwegzusatzmodul Injektionsereignisse und Vorgänge im Einweginjektor erfasst werden. Falls das Mehrwegzusatzmodul eine Kommunikationseinheit umfasst, können die generierten Messdaten an ein Anzeigegerät wie beispielsweise ein Smartphone des Benutzers übermittelt werden.

Das oben beschriebene System mit der Übertragungseinheit und der Ladeeinheit zum Laden des Energiespeichers mit dem Energieträger ist nicht nur für den erwähnten Einweginjektor und das Mehrwegzusatzmodul einsetzbar. Realisierbar sind auch nicht erfindungsgemäße Ausführungen bei denen der Energieträger auf einem Produktbehälter angeordnet ist und der Energiespeicher in einem Verabreichungssystem wie beispielswiese einem Injektionssystem oder einem Infusionssystem angeordnet ist.

Hierzu umfasst das Verabreichungssystem vorzugsweise eine Verabreichungseinheit zur dosierten Abgabe einer medizinischen Substanz und einen lösbar mit der Verabreichungseinheit verbindbaren Produktbehälter mit der medizinischen Substanz, wobei in einem verbundenen Zustand die medizinische Substanz aus dem Produktbehälter mit der Verabreichungseinheit verabreicht werden kann. Dabei ist das Verabreichungssystem dadurch gekennzeichnet dass der Produktbehälter ein Energieträger und eine Übertragungseinheit zum Übertragen von elektrischer Energie vom Energieträger umfasst und die Verabreichungseinheit einen aufladbaren Energiespeicher und eine Ladeeinheit zum Aufnehmen von elektrischer Energie für den Energiespeicher umfasst, wobei die Übertragungseinheit und die Ladeeinheit so ausgebildet sind, dass im verbundenen Zustand der Energiespeicher mit elektrischer Energie vom Energieträger aufladbar ist.

Der Energieträger des Produktbehälters speist die elektrischen Verbraucher in der Verabreichungseinheit während der Zeitdauer, in der der Produktbehälter mit der Verabreichungseinheit verbunden ist. Da für den Verabreichungsvorgang ein Produktbehälter in die Verabreichungseinheit eingesetzt werden muss, erfolgt automatisch und für den Anwender unbemerkt auch eine Ladung des Energiespeichers in der Verabreichungseinheit. Der Anwender muss den Ladungszustand des Energiespeichers nicht überwachen und auch nicht manuell eine Nachladung vornehmen. Das erhöht die Benutzerfreundlichkeit.

Wenn kein Produktbehälter mit der Verabreichungseinheit verbunden ist, wird üblicherweise die Verabreichungseinheit kaum genutzt. Meist wird sie in dieser Zeit nur gelagert und braucht daher gar keine oder nur sehr wenig elektrische Energie. Dadurch wird keine grosse Kapazität des Energiespeichers in der Verabreichungseinheit benötigt. Das ermöglicht den Einsatz eines Energiespeichers von kompakter Baugrösse, so dass die Verabreichungseinheit kompakt konstruiert werden kann.

Vorzugsweise ist die Verabreichungseinheit insgesamt eine Mehrwegeinheit, welche für einen oder mehrere Verabreichungsvorgänge zum Beispiel Injektionsvorgänge oder Infusionsvorgänge mit jeweils einem ausgetauschten Produktbehälter verwendbar ist. Die Verabreichungseinheit kann jedoch auch eine Mehrwegkomponente und eine Einwegkomponente umfassen. Die Mehrwegkomponente kann zum Beispiel eine Elektronik und einen Energiespeicher beinhalten, während die Einwegkomponente beispielsweise eine Kanüle und ein Kontaktelement zum Aufbringen auf der Haut umfassen kann. In einer bevorzugten Ausführung ist die Verabreichungseinheit als Injektor ausgebildet, insbesondere als Mehrweginjektor. In einer alternativen Ausführung kann die Verabreichungseinheit jedoch auch eine medizinische Pumpe, beispielsweise eine Insulinpumpe sein.

Der Produktbehälter ist vorzugsweise ein Karpule, in der die medizinische Substanz, insbesondere ein flüssiges Medikament, gelagert werden kann. Bevorzugt handelt es sich bei der Karpule um ein zylindrisches Gefäss. In einer bevorzugten Ausführung ist die Verabreichungseinheit als Injektor ausgebildet und der Produktbehälter als eine in den Injektor einsetzbare und aus diesem durch den Patienten wieder entfernbare Karpule realisiert.

Der Energiespeicher in der Verabreichungseinheit ist vorzugsweise als Akkumulator ausgebildet. Bevorzugt ist der Energieträger eine Einwegbatterie, welche in einem einmaligen Vorgang entladbar ist. Vorzugsweise ist der Energieträger auf einer Aussenseite des Produktbehälters angeordnet. In einer anderen Ausführung kann der Energieträger jedoch auch in den Produktbehälter, insbesondere in eine Wandung des Produktbehälters, integriert sein.

In einer bevorzugten Ausführung ist der Energiespeicher als sogenannte grüne, gedruckte Batterie ausgeführt, welche keine spezielle Entsorgung benötigt. Das ist benutzerfreundlich und erleichtert die Handhabung, da die Karpule auf üblichem Wege zusammen mit der Batterie entsorgt werden kann. Vorzugsweise muss nach dem Gebrauch die Batterie also nicht von der Karpule getrennt werden.

In einer Ausführung kann das Verabreichungssystem neben der Übertragung von elektrischer Energie zwischen dem Produktbehälter und der Verabreichungseinheit auch dazu genutzt werden, Signale zwischen dem Produktbehälter und der Verabreichungseinheit zu übermitteln, um eine Datenübertragung zu ermöglichen. Hierzu umfassen der Produktbehälter und die Verabreichungseinheit je eine Kommunikationsschnittstelle. So können zum Beispiel mittels der Signale Informationen zur medizinischen Substanz im Produktebehälter, Informationen über das Herstell- oder Verfallsdatum oder zum Füllstand des Produktebehälters über die Kommunikationsschnittstelle vom Produktebehälter zur Verabreichungseinheit übermittelt werden. Das Übertragen der Signale kann beispielsweise mittels "radio-frequency identification" (RFID) erfolgen. In diesem Fall umfasst der Produktbehälter einen RFID-Transponder, der einen Code enthält und die Verabreichungseinheit umfasst ein Lesegerät zum Auslesen des Codes.

Vorzugsweise umfasst die Übertragungseinheit des Produktbehälters eine erste Kontaktplatte und die Ladeeinheit der Verabreichungseinheit eine zweite Kontaktplatte zum elektrischen Verbinden mit der ersten Kontaktplatte. Dabei ist die erste Kontaktplatte elektrisch mit dem Energieträger und die zweite Kontaktplatte elektrisch mit dem Energiespeicher verbunden, so dass elektrische Energie vom Energieträger über die erste und die zweite Kontaktplatte zum aufladbaren Energiespeicher in der Verabreichungseinheit übertragbar ist, wenn ein Produktbehälter mit der Verabreichungseinheit verbunden ist. Das bedeutet, in diesem verbundenen Zustand berührt die erste Kontaktplatte die zweite Kontaktplatte. Eine solche elektrische Verbindung ist konstruktiv einfach realisierbar und ermöglicht zudem eine zuverlässige elektrische Kontaktierung

Die Verabreichungseinheit zur dosierten Abgabe einer medizinischen Substanz und zur Aufnahme eines Produktbehälters mit der medizinischen Substanz umfasst ein Energiespeicher und eine Aufnahmevorrichtung zum Aufnehmen des Produktbehälters. Die Verabreichungseinheit ist dadurch gekennzeichnet, dass die Aufnahmevorrichtung eine Innenwand umfasst, an der eine Ladeeinheit angeordnet ist zum Aufnehmen von elektrischer Energie von einem am Produktbehälter angeordneten Energieträger, wobei die Ladeeinheit mit einer Übertragungseinheit des Produktbehälters zusammenwirkt, wenn der Produktbehälter mit der Verabreichungseinheit verbunden ist.

In einer bevorzugten Ausführung ist die Aufnahmevorrichtung der Verabreichungseinheit als Hohlraum ausgebildet, in den der Produktbehälter eingesetzt und mit der Verabreichungseinheit verbunden werden kann, wobei bei eingesetztem Produktbehälter die Übertragungseinheit mit der Ladeeinheit der Verabreichungseinheit zusammenwirkt.

Bevorzug ist der Produktbehälter zum Aufbewahren einer medizinischen Substanz und zum lösbaren Verbinden mit einer Verabreichungseinheit, dadurch gekennzeichnet, dass der Produktbehälter einen Energieträger und eine Übertragungseinheit zum Übertragen von elektrischer Energie zur Verabreichungseinheit umfasst.

Bevorzugt ist die Übertragungseinheit entlang eines Umfangs des Produktbehälters angeordnet. Dabei ist der Energieträger im Produktbehälter vorzugsweise als gedruckte Batterie ausgebildet. In einer bevorzugten Ausführung weist die gedruckte Batterie eine gekrümmte Form auf. Dadurch kann die gedruckte Batterie in platzsparender Weise auf einer gekrümmten Aussenfläche, insbesondere auf einer zylindrischen Oberfläche des Produktbehälters angebracht werden.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt einen Einweginjektor;
- Fig. 2: zeigt ein Mehrwegzusatzmodul;
- Fig. 3: zeigt ein Injektionssystem mit dem Einweginjektor im Mehrwegzusatzmodul;
- Fig. 4: zeigt schematisch eine Schnittansicht des Injektionssystems;
- Fig. 5: zeigt eine weitere Ausführung des Injektionssystems, bei der das Mehrwegzusatzmodul mehrere Kontaktplatten umfasst;
- Fig. 6: zeigt eine Frontansicht des Mehrwegzusatzmoduls aus Fig. 5;
- Fig. 7: zeigt eine Ausführung des Injektionssystems bei der die Übertragungseinheit am Injektor stirnseitig angeordnet ist; und
- Fig. 8: zeigt eine weitere Ausführung des Mehrwegzusatzmoduls, welches hülsenförmig ausgebildet ist.

### FIGURENBESCHREIBUNG

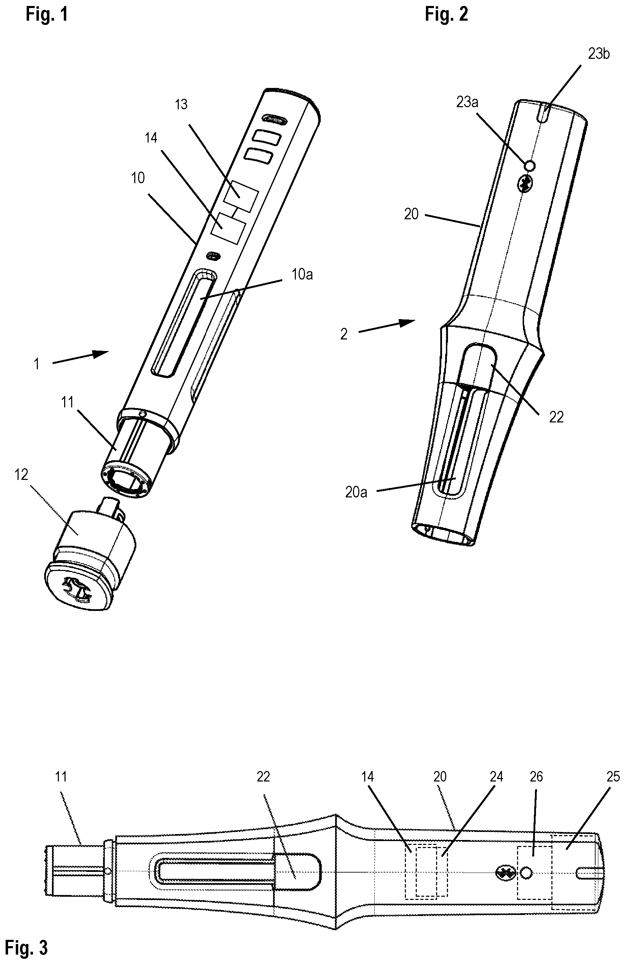

Figur 1 zeigt einen Einweginjektor in der Form eines Autoinjektors 1 für ein Injektionssystem. Figur 2 stellt ein Mehrwegzusatzmodul 2 des Injektionssystems dar und in Figur 3 ist das gesamte Injektionssystem mit dem Autoinjektor 1 und dem mit dem Autoinjektor 1 verbundenen Mehrwegzusatzmodul 2 ersichtlich.

Wie in Figur 1 erkennbar ist, umfasst der Autoinjektor 1 ein längliches, um eine Längsachse symmetrisches Gerätegehäuse 10 und eine Nadelschutzhülse 11, welche zwischen einer ersten, eine Nadel des Autoinjektors 1 abschirmenden Position und einer zweiten, die Nadel freigebenden Position verschiebbar ist. Ein Nadelschutzkappenentferner 12 ist im Auslieferungszustand auf ein dem einstechseitigen Ende gegenüberliegendes Ende des Autoinjektors 1 montiert und muss vor der Injektion zusammen mit einer die Nadel mechanisch schützenden Nadelschutzkappe entfernt werden. Weiter umfasst der Autoinjektor 1 einen Energieträger, welcher als auf einer Aussenseite des Gerätegehäuses 10 angebrachte gedruckte Einwegbatterie 13 ausgebildet ist und eine Übertragungseinheit 14, die mit einer Verbindungsleitung elektrisch mit der Einwegbatterie 13 verbunden ist.

Das in Figur 2 dargestellte Mehrwegzusatzmodul 2 hat ein längliches, hülsenförmiges Modulgehäuse 20 mit einem Hohlraum als Aufnahme für einen Autoinjektor 1, wobei die Aufnahme an die im Querschnitt quadratische Form des Gerätegehäuses 10 abgestimmt ist, so dass zum Verbinden der Autoinjektor 1 in das Mehrwegzusatzmodul 2 eingeschoben werden kann. Das Mehrwegzusatzmodul 2 weist einen Löseknopf 22, eine optische Zustandsanzeige 23a und eine Verbindungsanzeige 23b für eine visuelle Rückmeldung an den Benutzer, und seitliche Öffnungen 20a auf, welche im verbundenen Zustand mit Fenstern 10a im Gerätegehäuse 10 ausgerichtet sind und den Blick auf eine im Autoinjektor 1 gelagerte Substanz ermöglichen. Alternativ kann das Mehrwegzusatzmodul 2 auch deutlich kürzer sein als der Autoinjektor 1, also zum Beispiel nur halb so lang wie der Autoinjektor 1, ausgeführt sein. In einem hinteren dem Eingang des Hohlraums abgewandten Bereich umfasst das Mehrwegzusatzmodul 2 einen Lithium-Ionen-Akkumulator 25 als Energiespeicher 27, eine Ladeeinheit 24, welche elektrisch mit dem Akkumulator 25 verbunden ist, und elektrische Verbraucher 26 wie einen Kraftsensor, eine Auswertelektronik zur Signalverarbeitung und Datenspeicherung und eine Kommunikationseinheit zur drahtlosen Kommunikation mit einem mobilen Gerät (nicht dargestellt).

In Figur 3 sind die Positionen der Übertragungseinheit 14 des Autoinjektors 1 und der Kontaktplatte 24 des Mehrwegzusatzmoduls 2 ersichtlich, dargestellt in gestrichelten Linien. Im gezeigten verbundenen Zustand liegen die Übertragungseinheit 14 und die Ladeeinheit 24 in Längsrichtung im Wesentlichen übereinander und berühren sich, so dass eine elektrische Verbindung zwischen der Einwegbatterie 13 und dem Akkumulator 25 hergestellt wird.

Vor einem Injektionsvorgang wird das Mehrwegzusatzmodul 2 mit dem Autoinjektor 1 verbunden, indem das Mehrwegzusatzmodul 2 von einem dem einstechseitiges Ende gegenüberliegenden Ende des Autoinjektors 1 in Richtung der Längsachse auf den Autoinjektor 1 aufgeschoben wird. Wenn das Gerätegehäuse 10 vollständig bis zu einem Anschlag im Hohlraum des Modulgehäuses 20 eingeschoben ist, befindet sich das Injektionssystem im verbundenen Zustand. In diesem ist der Autoinjektor 1 mechanisch im Modulgehäuse 20 gehalten, indem eine Einrastfeder im Modulgehäuse 20 in einer Kerbe im Gerätegehäuse 10 einrastet. Über den Löseknopf 22 kann die Einrastfeder gelöst werden und der Autoinjektor 1 kann aus dem Mehrwegzusatzmodul 2 in axialer Richtung herausgleiten.

Da sich im verbundenen Zustand die Ladeeinheit 24 des Mehrwegzusatzmodul 2 die Übertragungseinheit 14 des Autoinjektors 1 berührt, besteht eine sichere elektrische Verbindung zwischen der Einwegbatterie 13 und dem Akkumulator 25. Somit kann im verbundenen Zustand der Akkumulator 25 durch die Einwegbatterie 13 aufgeladen werden. Zudem werden die elektrischen Verbraucher 26, wie der Sensor, die Auswertelektronik und die Kommunikationseinheit im Mehrwegzusatzmodul 2 über den Akkumulator 25 gespeist, welcher laufend mit elektrischer Energie von der Einwegbatterie 13 versorgt wird. Wenn das Mehrwegzusatzmodul 2 nicht mit dem Autoinjektor 1 verbunden ist, beispielsweise während dem Auswechseln des Autoinjektors 1, versorgt der Akkumulator 25 die elektrischen Verbraucher 26 mit der im Akkumulator gespeicherten elektrischen Energie. Wird das Mehrwegzusatzmodul 2 wieder mit einem Autoinjektor 1 verbunden, wird der Akkumulator 25 wieder mit elektrischer Energie aufgeladen. Zu Energiesparzwecken kann das Mehrwegzusatzmodul 2 einen Stromsparmodus aufweisen, der aktiviert wird, sobald der Akkumulator 25 nicht mehr mit der Einwegbatterie 13 verbunden ist. Wenn wieder ein Autoinjektor 1 mit dem Mehrwegzusatzmodul 2 verbunden wird, kann entsprechend der Stromsparmodus deaktiviert werden.

Zu Beginn einer Injektion wird das einstechseitige Ende des Autoinjektors 1, also die Nadelschutzhülse 11, durch den Benutzer auf die Einstechstelle gedrückt, so dass die Nadelschutzhülse 11 unter Spannung einer Nadelschutzhülsenfeder in den Autoinjektor 1 eingeschoben wird und gleichzeitig die Nadel in die Einstechstelle eindringt. Während des Injektionsvorgangs und im verbundenen Zustand kann der Kraftsensor eine ausgeübte axiale Kraftkomponente in Richtung der Längsachse des Autoinjektors 1 messen. Anhand dieser Messungen kann die Auswertelektronik den Injektionsbeginn und -abschluss erkennen. Die Kommunikationseinheit überträgt die erfassten Injektionsinformationen drahtlos an ein kompatibles mobiles Gerät, beispielsweise an ein Smartphone mit einer speziellen Applikation, oder an einen entsprechend konfigurierten tragbaren Computer. Zusätzlich kann das Mehrwegzusatzmodul 2 weitere Sensoren wie beispielsweise einen Lagesensor, einen Temperatursensor, einen kapazitiven oder induktiven Näherungssensor oder ein Mikrophon zur Erkennung eines Startklicks einer Injektion umfassen.

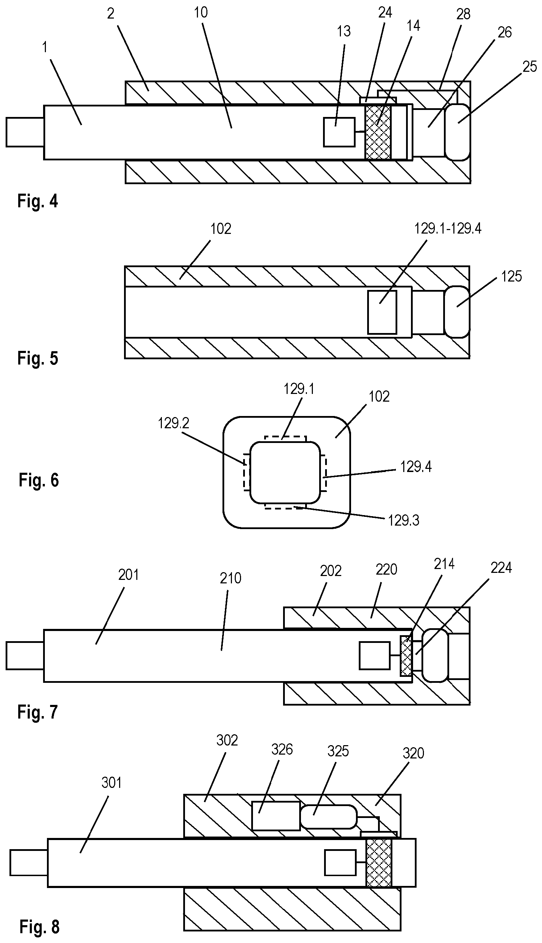

Figur 4 stellt schematisch eine Schnittansicht des erfindungsgemässen Injektionssystems mit dem Autoinjektor 1 und dem verbundenen Mehrwegzusatzmodul 2 dar, wobei der Schnitt durch die Längsachse des Mehrwegzusatzmoduls 2 verläuft. Der Autoinjektor 1 ist nicht geschnitten dargestellt. Ersichtlich ist in dieser Darstellung die auf einer Innenfläche des Hohlraums des Modulgehäuses 20 angeordnete Ladeeinheit 24, die in Kontakt mit der Übertragungseinheit 14 des Autoinjektors 1 steht. Die Ladeeinheit 24 ist über eine Verbindungsleitung 28 elektrisch mit dem Akkumulator 25 verbunden, welcher in Längsrichtung hinter den elektrischen Verbrauchen 26 platziert ist und diese mit elektrischer Energie speist. Die Ladeeinheit 24 des Mehrwegzusatzmoduls 2 ist dabei in einem hinteren, dem Eingang des Hohlraums abgewandten Endbereich des Hohlraums angeordnet. Damit eine physische Kontaktierung zwischen Übertragungseinheit 14 und Ladeeinheit 24 sichergestellt werden kann, ist die Ladeeinheit 24 vorzugsweise als federnde Kontaktplatte ausgebildet, die leicht in den Hohlraum des Modulgehäuses 20 hineinragt. Wenn der Autoinjektor 1 in den Hohlraum eingeführt wird, wird die federnde Kontaktplatte leicht zurückgebogen. Dadurch ist die Kontaktplatte stets in physischem Kontakt mit der Übertragungseinheit 14, wenn der Autoinjektor 1 mit dem Mehrwegzusatzmodul 2 verbunden ist.

Die Übertragungseinheit 14 des Autoinjektors 1 kann über den ganzen Umfang des Autoinjektors 1 angeordnet sein. Vorzugsweise umfasst die Übertragungseinheit vier Kontaktplatten, die je auf einer Seite des Autoinjektors 1 angeordnet sind. In diesem Fall muss der Autoinjektor 1 nicht in einer bestimmten Ausrichtung in den Hohlraum eingeführt werden, da unabhängig von der Ausrichtung des Autoinjektors 1 mindestens eine der Kontaktplatten mit der Ladeeinheit 24 in Berührung kommt und dadurch eine elektrische Verbindung herstellen kann.

Figur 5 zeigt schematisch eine weitere Ausführung des Mehrwegzusatzmoduls 102 in der Schnittansicht ohne verbundenen Autoinjektor 1. Figur 6 stellt schematisch eine Frontansicht dieser weiteren Ausführung des Mehrwegzusatzmoduls 102 dar. Die in den Figuren 5 und 6 gezeigte Ausführung weist im Unterschied zur Ausführung in Figur 4 eine Ladeeinheit 124 auf, welche vier Kontaktplatten 129.1 -129.4 umfasst, wobei an jeder der vier Innenflächen des Hohlraums eine Kontaktplatte 129.1 - 129.4 angeordnet ist, ersichtlich in Figur 6. Alle vier Kontaktplatten 129.1 - 129.4 sind über Verbindungsleitungen (in den Figuren 5 und 6 nicht dargestellt) mit dem Akkumulator 125 verbunden. Bei dieser Ausführung kann die Übertragungseinheit 114 nur auf einer Seitenfläche des Autoinjektors 1 angeordnet sein, da diese unabhängig von der Ausrichtung des Autoinjektors 1 mit einer der vier Kontaktplatten 129.1 - 129.4 in Kontakt kommt. In einer alternativen Ausführung, in der der Hohlraum im Mehrwegzusatzmodul 202 nicht einen quadratischen sondern einen kreisrunden Querschnitt aufweist, können die Kontaktplatten eine gekrümmte Form aufweisen, so dass sie ebenfalls entlang des Umfangs an den Innenflächen des Hohlraums angeordnet werden können. Zudem können die Kontaktplatten entsprechend auch bei einem polygonförmigen Querschnitt des Hohlraums an den Innenflächen des Hohlraums angeordnet sein.

Eine weitere Ausführung des Injektionssystems ist schematisch in Figur 7 gezeigt. Die Übertragungseinheit 214 des Autoinjektors 201 ist in dieser Ausführung stirnseitig an einer Abschlussfläche des Autoinjektors 201, welche rechtwinklig zur Längsachse ausgerichtet ist, angebracht. Entsprechend ist die Ladeeinheit 224 des Mehrwegzusatzmodul 202 an einer Bodenfläche des Hohlraums angeordnet, so dass die Übertragungseinheit 214 des Autoinjektors 201 die Ladeeinheit 224 des Mehrwegzusatzmodul 202 kontaktiert, wenn der Autoinjektor 201 in Richtung der Längsachse vollständig in den Hohlraum eingeschoben ist. Zudem ist das Modulgehäuse 220 in der in Figur 7 gezeigten Ausführung kürzer ausgestaltet als bei den Ausführungen in Figur 1 - 5. Für die Injektion hält der Benutzer mit seiner Hand das Injektionssystem nicht am Mehrwegzusatzmodul 202 sondern am Gerätegehäuse 210 des Autoinjektors 201 fest. Alternativ kann auch bei dieser Ausführung das Modulgehäuse 220 länger ausgebildet sein, so dass es einen Griff umfasst zum Halten des Injektionssystems am Modulgehäuse 220.

In Figur 8 ist eine weitere Ausführung des Mehrwegzusatzmoduls 302 dargestellt, bei der das Mehrwegzusatzmodul 302 hülsenförmig ausgebildet ist. Wenn das Mehrwegzusatzmodul 302 mit dem Autoinjektor 301 verbunden ist, bleibt das dem einstechseitigen Ende gegenüberliegende hintere Ende des Autoinjektors 301 frei. Damit beim Verbinden das Mehrwegzusatzmodul 302 in axialer Richtung zum Autoinjektor 301 einfach positionierbar ist, weist das Mehrwegzusatzmodul 302 vorzugsweise ein Rastelement auf, welches in eine Aussparung im Autoinjektor 301 einrastet, wenn das Mehrwegzusatzmodul 302 in axialer Richtung relativ zum Autoinjektor 301 korrekt positioniert ist. Diese Ausführung des Mehrwegzusatzmoduls 302 ist insbesondere für Autoinjektoren 301 vorteilhaft, bei denen am hinteren Ende des Autoinjektors 301 mit einem Einstellrad Einstellungen vorgenommen werden können und dadurch das hintere Ende für den Benutzer frei zugänglich sein muss. Der Akkumulator 325 und die elektrischen Verbraucher 326 sind bei dieser Ausführung seitlich des Autoinjektors 301 im Modulgehäuse 320 untergebracht.

Alternativ zu den in den Figuren 1 - 8 dargestellten Ausführungen besteht auch die Möglichkeit, dass die gedruckte Einwegbatterie in Längsrichtung weiter von der Übertragungseinheit des Autoinjektors beabstandet ist. So kann beispielsweise die Batterie in einem Bereich des einstechseitigen Endes des Autoinjektors angeordnet sein und über eine entsprechende Verbindungsleitung mit der Übertragungseinheit verbunden sein, welche in einem dem einstechseitigen Ende gegenüberliegenden Ende des Autoinjektors angeordnet ist. Bei einer solchen Ausführung befindet sich im verbundenen Zustand die Batterie nicht innerhalb des Hohlraums. In einer weiteren Ausführung besteht auch die Möglichkeit, dass sowohl die Batterie wie auch die Übertragungseinheit des Autoinjektors im Bereich des einstechseitigen Endes angebracht sind. Entsprechend muss in diesem Fall die Ladeeinheit des Mehrwegzusatzmoduls im Bereich des Eingangs des Hohlraumes angeordnet sein, so dass sich im verbundenen Zustand die Übertragungseinheit des Autoinjektors in Längsrichtung mit der Ladeeinheit des Mehrwegzusatzmoduls überlappt und berührt.

In einer weiteren nicht erfindungsgemässen Ausführung besteht auch die Möglichkeit, dass ein Mehrweginjektor eine lösbar mit dem Mehrweginjektor verbindbare Karpule mit einer medizinischen Substanz umfasst. Dabei kann die Karpule eine gedruckte Batterie umfassen, welche einen Energiespeicher im Mehrweginjektor auflädt. Die Karpule wird nach dem Ausschütten zusammen mit der gedruckten Batterie ausgewechselt. Vorzugsweise ist die Batterie eine sogenannte "grüne Batterie", welche mit dem normalen Hausmüll entsorgt werden kann.

In einer anderen nicht erfindungsgemässen Ausführung kann die gedruckte Batterie auch direkt auf einem Einweginjektor aufgebracht sein, um eine Kommunikationseinheit des Einweginjektors mit elektrischer Energie zu versorgen. Das bedeutet, die gedruckte Batterie wird nur benötigt, um Daten vom Einweginjektor an einen Empfänger, zum Beispiel ein kompatibles mobiles Gerät, wie ein Smartphone oder einen tragbaren Computer zu senden. In diesem Fall genügt bereits eine gedruckte Batterie, welche eine Fläche zwischen 3 mm² und 8 mm² aufweist. Bevorzugt wird auch in diesem Fall eine grüne Batterie verwendet, welche keine spezielle Entsorgung bedingt.

### BEZUGSZEICHENLISTE

- 1, 201, 301: Autoinjektor
- 10, 210: Gerätegehäuse
- 10a: Fenster
- 11: Nadelschutzhülse
- 12: Nadelschutzkappenentferner
- 13: Einwegbatterie
- 14, 14, 214: Übertragungseinheit
- 129.1 - 129.4: Kontaktplatten
- 2, 102, 202, 302: Mehrwegzusatzmodul
- 20, 220, 320: Modulgehäuse
- 20a: Öffnung
- 22: Löseknopf
- 23a: Zustandsanzeige
- 23b: Verbindungsanzeige
- 24, 124, 224: Ladeeinheit
- 25, 125, 325: Akkumulator
- 26, 326: Elektrische Verbraucher
- 27: Energiespeicher
- 28: Verbindungsleitung

## Patentansprüche

1. Injektionssystem umfassend einen Einweginjektor (1) zur dosierten Abgabe einer medizinischen Substanz an einen Patienten und ein lösbar mit dem Einweginjektor (1) verbindbares Mehrwegzusatzmodul (2), welches in einem verbundenen Zustand Injektionsereignisse des Einweginjektors (1) erkennen kann, wobei der Einweginjektor (1) ein Autoinjektor ist, welcher entlang einer Längsachse eine längliche Form aufweist, eine Einwegbatterie (13) und eine Übertragungseinheit (14) zum Übertragen von elektrischer Energie von der Einwegbatterie (13) umfasst, wobei das Mehrwegzusatzmodul (2) hülsenförmig ausgebildet ist und eine Öffnung aufweist, in die mindestens ein Bereich des Autoinjektors in Richtung der Längsachse eingeschoben werden kann, so dass das Mehrwegzusatzmodul (2) im verbundenen Zustand den Autoinjektor teilweise umgibt und das Mehrwegzusatzmodul (2) weiter einen aufladbaren Energiespeicher (25) und eine Ladeeinheit zum Aufnehmen von elektrischer Energie für den Energiespeicher (25) umfasst, wobei die Übertragungseinheit (14) und die Ladeeinheit (24) so ausgebildet sind, dass im verbundenen Zustand der Energiespeicher (25) mit elektrischen Energie von der Einwegbatterie (13) aufladbar ist.

2. Injektionssystem nach Anspruch 1, **dadurch gekennzeichnet, dass** die Übertragungseinheit (14) des Autoinjektors (1) eine erste Kontaktplatte (14) und die Ladeeinheit (24) des Mehrwegzusatzmoduls eine zweite Kontaktplatte (24) zum elektrischen Verbinden mit der ersten Kontaktplatte (14) umfasst.

3. Injektionssystem nach Anspruch 1, **dadurch gekennzeichnet, dass** die Übertragungseinheit (14) einen Oszillator und eine erste Spule umfasst zum Erzeugen eines magnetischen Wechselfeldes und die Ladeeinheit (24) eine zweite Spule umfasst, wobei die Übertragungseinheit (14) und die Ladeeinheit (24) dazu ausgebildet sind eine kontaktlose und induktive Übertragung der elektrischen Energie von der Einwegbatterie (13) zum aufladbaren Energiespeicher (25) zu ermöglichen.

4. Injektionssystem nach Anspruch 1, **dadurch gekennzeichnet, dass** die Übertragungseinheit (14) einen Oszillator und ein erstes Element und die Ladeeinheit (24) ein zweites Element umfasst, wobei das erste und das zweite Element einen Kondensator bilden für eine kontaktlose und kapazitive Übertragung der elektrischen Energie von der Einwegbatterie (13) zum aufladbaren Energiespeicher (25).

5. Injektionssystem nach einem der Ansprüche 1 bis 4, **dadurch gekennzeichnet, dass** der Autoinjektor (1) ein um die Längsachse symmetrisches Gerätegehäuse (10) aufweist.

6. Injektionssystem nach einem der Ansprüche 1 bis 5, **dadurch gekennzeichnet, dass** die Ladeeinheit (24) entlang einer Innenfläche des Mehrwegzusatzmoduls (2) angeordnet ist.

7. Injektionssystem nach einem der Ansprüche 1 bis 5, **dadurch gekennzeichnet, dass** die Übertragungseinheit (14) entlang eines Umfangs des Einweginjektors (1) angeordnet ist.

8. Einweginjektor (1) zur dosierten Abgabe einer Substanz an einen Patienten, wobei der Einweginjektor (1) lösbar mit einem Mehrwegzusatzmodul (2) verbindbar ist, wobei der Einweginjektor (1) ein Autoinjektor ist, welcher entlang einer Längsachse eine längliche Form aufweist und eine Einwegbatterie (13) und eine Übertragungseinheit (14) zum Übertragen von elektrischer Energie von der Einwegbatterie (13) umfasst, wobei die Übertragungseinheit (14) so ausgebildet ist, dass in einem verbundenen Zustand die elektrische Energie von der Einwegbatterie (13) zu einem aufladbaren Energiespeicher (25) im Mehrwegzusatzmodul (2) übertragbar ist zum Aufladen des Energiespeichers (25).

9. Einweginjektor (1) nach Anspruch 8, **dadurch gekennzeichnet, dass** die Einwegbatterie (13) auf einer Aussenfläche des Einweginjektors (1) angeordnet ist.

10. Einweginjektor (1) nach einem der Ansprüche 8 oder 9, **dadurch gekennzeichnet, dass** die Einwegbatterie (13) als gedruckte Batterie ausgebildet ist.

11. Einweginjektor (1) nach Anspruch 10, **dadurch gekennzeichnet, dass** die gedruckte Batterie eine gekrümmte Form aufweist.

12. Einweginjektor (1) nach Anspruch 11, **dadurch gekennzeichnet, dass** die gedruckte Batterie eine grüne Batterie ist, welche aus Materialien gefertigt ist, welche keine spezielle Entsorgung benötigen und wobei die Batterie vorzugsweise eine Grösse zwischen 120 mm² und 200 mm² aufweist.

## Claims

1. Injection system comprising a disposable injector (1) for the dosed administration of a medical substance to a patient, and a reusable add-on module (2) which can be detachably connected to the disposable injector (1) and which, in a connected state, can detect injection events of the disposable injector (1), wherein
the disposable injector (1) is an autoinjector having an elongate shape along a longitudinal axis, and comprises a disposable battery (13) and a transmission unit (14) for transmitting electrical energy from the disposable battery (13), wherein the reusable add-on module (2) is sleeve-shaped and has an opening into which at least a region of the autoinjector can be inserted in the direction of the longitudinal axis so that the reusable add-on module (2) partially surrounds the autoinjector in the connected state, and the reusable add-on module (2) further comprises a chargeable energy storage device (25) and a charging unit for receiving electrical energy for the energy storage device (25), wherein the transmission unit (14) and the charging unit (24) are designed such that, in the connected state, the energy storage device (25) can be charged with electrical energy from the disposable battery (13).

2. Injection system according to claim 1, **characterized in that** the transmission unit (14) of the autoinjector (1) comprises a first contact plate (14) and the charging unit (24) of the reusable add-on module comprises a second contact plate (24) for electrical connection with the first contact plate (14).

3. Injection system according to claim 1, **characterized in that** the transmission unit (14) comprises an oscillator and a first coil for generating an alternating magnetic field and the charging unit (24) comprises a second coil, the transmission unit (14) and the charging unit (24) being designed to enable contactless and inductive transmission of the electrical energy from the disposable battery (13) to the chargeable energy storage device (25).

4. Injection system according to claim 1, **characterized in that** the transmission unit (14) comprises an oscillator and a first element and the charging unit (24) comprises a second element, the first and the second element forming a capacitor for contactless and capacitive transmission of the electrical energy from the disposable battery (13) to the chargeable energy storage device (25).

5. Injection system according to any of claims 1 to 4, **characterized in that** the autoinjector (1) has a device housing (10) that is symmetrical about the longitudinal axis.

6. Injection system according to any of claims 1 to 5, **characterized in that** the charging unit (24) is arranged along an inner surface of the reusable add-on module (2).

7. Injection system according to any of claims 1 to 5, **characterized in that** the transmission unit (14) is arranged along a circumference of the disposable injector (1).

8. Disposable injector (1) for the dosed administration of a substance to a patient, wherein the disposable injector (1) can be detachably connected to a reusable add-on module (2), wherein the disposable injector (1) is an autoinjector which has an elongate shape along a longitudinal axis and comprises a disposable battery (13) and a transmission unit (14) for transmitting electrical energy from the disposable battery (13), wherein the transmission unit (14) is designed such that, in a connected state, the electrical energy can be transmitted from the disposable battery (13) to a chargeable energy storage device (25) in the reusable add-on module (2) in order to charge the energy storage device (25).

9. Disposable injector (1) according to claim 8, **characterized in that** the disposable battery (13) is arranged on an outer surface of the disposable injector (1).

10. Disposable injector (1) according to either of claims 8 or 9,
**characterized in that** the disposable battery (13) is designed as a printed battery.

11. Disposable injector (1) according to claim 10, **characterized in that** the printed battery has a curved shape.

12. Disposable injector (1) according to claim 11, **characterized in that** the printed battery is a green battery that is made from materials which do not require special disposal, the battery preferably being between 120 mm² and 200 mm² in size.

## Revendications

1. Système d'injection comprenant un injecteur jetable (1) pour l'administration dosée d'une substance médicale à un patient et un module complémentaire réutilisable (2) pouvant être relié de manière amovible à l'injecteur jetable (1), qui peut reconnaître, dans un état relié, des événements d'injection de l'injecteur jetable (1), dans lequel
l'injecteur jetable (1) est un auto-injecteur qui présente une forme allongée le long d'un axe longitudinal, comprend une batterie jetable (13) et une unité de transmission (14) pour transmettre de l'énergie électrique à partir de la batterie jetable (13), dans lequel le module complémentaire réutilisable (2) est conçu en forme de douille et présente une ouverture dans laquelle au moins une zone de l'auto-injecteur peut être insérée dans la direction de l'axe longitudinal, de sorte qu'à l'état relié, le module complémentaire réutilisable (2) entoure partiellement l'auto-injecteur et le module complémentaire réutilisable (2) comprend en outre un accumulateur d'énergie (25) rechargeable et une unité de charge pour recevoir de l'énergie électrique pour l'accumulateur d'énergie (25), dans lequel l'unité de transmission (14) et l'unité de charge (24) sont conçues de telle sorte qu'à l'état relié, l'accumulateur d'énergie (25) peut être chargé avec de l'énergie électrique provenant de la batterie jetable (13).

2. Système d'injection selon la revendication 1, **caractérisé en ce que** l'unité de transmission (14) de l'auto-injecteur (1) comprend une première plaque de contact (14) et l'unité de charge (24) du module complémentaire réutilisable comprend une seconde plaque de contact (24) destinée à être connectée électriquement à la première plaque de contact (14).

3. Système d'injection selon la revendication 1, **caractérisé en ce que** l'unité de transmission (14) comprend un oscillateur et une première bobine pour générer un champ magnétique alternatif et l'unité de charge (24) comprend une seconde bobine, dans lequel l'unité de transmission (14) et l'unité de charge (24) sont conçues pour permettre une transmission sans contact et inductive de l'énergie électrique de la batterie jetable (13) à l'accumulateur d'énergie (25) rechargeable.

4. Système d'injection selon la revendication 1, **caractérisé en ce que** l'unité de transmission (14) comprend un oscillateur et un premier élément et l'unité de charge (24) comprend un second élément, dans lequel les premier et second éléments forment un condensateur pour une transmission sans contact et capacitive de l'énergie électrique de la batterie jetable (13) à l'accumulateur d'énergie (25) rechargeable.

5. Système d'injection selon l'une des revendications 1 à 4,
**caractérisé en ce que** l'auto-injecteur (1) présente un boîtier d'appareil (10) symétrique autour de l'axe longitudinal.

6. Système d'injection selon l'une des revendications 1 à 5,
**caractérisé en ce que** l'unité de charge (24) est agencée le long d'une surface interne du module complémentaire réutilisable (2).

7. Système d'injection selon l'une des revendications 1 à 5,
**caractérisé en ce que** l'unité de transmission (14) est agencée le long d'une périphérie de l'injecteur jetable (1).

8. Injecteur jetable (1) pour l'administration dosée d'une substance à un patient, dans lequel l'injecteur jetable (1) peut être relié de manière amovible à un module complémentaire réutilisable (2), dans lequel l'injecteur jetable (1) est un auto-injecteur qui présente une forme allongée le long d'un axe longitudinal et comprend une batterie jetable (13) et une unité de transmission (14) pour transmettre l'énergie électrique de la batterie jetable (13), dans lequel l'unité de transmission (14) est conçue de telle sorte que, dans un état relié, l'énergie électrique peut être transmise de la batterie jetable (13) à un accumulateur d'énergie (25) rechargeable dans le module complémentaire réutilisable (2) pour charger l'accumulateur d'énergie (25).

9. Injecteur jetable (1) selon la revendication 8, **caractérisé en ce que** la batterie jetable (13) est agencée sur une surface externe de l'injecteur jetable (1).

10. Injecteur jetable (1) selon l'une des revendications 8 ou 9,
**caractérisé en ce que** la batterie jetable (13) est conçue comme une batterie imprimée.

11. Injecteur jetable (1) selon la revendication 10, **caractérisé en ce que** la batterie imprimée présente une forme incurvée.

12. Injecteur jetable (1) selon la revendication 11, **caractérisé en ce que** la batterie imprimée est une batterie verte qui est fabriquée à partir de matériaux qui ne nécessitent pas d'élimination spéciale et dans lequel la batterie présente de préférence une taille comprise entre 120 mm² et 200 mm².
